# EUROPÄISCHE PATENTSCHRIFT

(11) **EP 3 359 961 B1**
(45) Veröffentlichungstag und Bekanntmachung des Hinweises auf die Patenterteilung: **05.08.2020**
(21) Anmeldenummer: 15785049.6
(22) Anmeldetag: 09.10.2015
(51) Int. Cl.: G01N 33/49, B01L 3/00

(54) **VERFAHREN ZUR PHASENTRENNUNG EINES FLÜSSIGEN GEMISCHS UND NACHFOLGENDER SEPARIERUNG EINES FLÜSSIGEN ÜBERSTANDS UND PHASENSEPARATOR FÜR DAS VERFAHREN**
METHOD FOR SEPARATING THE PHASES OF A LIQUID MIXTURE AND SUBSEQUENTLY SEPARATING A LIQUID SUPERNATANT AND PHASE SEPARATOR FOR THE METHOD
PROCÉDÉ POUR LA SÉPARATION DE PHASE D'UN MÉLANGE LIQUIDE ET POUR LA SÉPARATION CONSÉCUTIVE D'UN SURNAGEANT LIQUIDE ET SÉPARATEUR DE PHASE POUR LE PROCÉDÉ

(43) Veröffentlichungstag der Anmeldung: 15.08.2018
(73) Patentinhaber: Scheiter, Oliver, 59387 Ascheberg (DE)
(72) Erfinder: Scheiter, Oliver, 59387 Ascheberg (DE)
(74) Vertreter: Kayser, Christoph
(86) Internationale Anmeldenummer: PCT/DE2015/000493
(87) Internationale Veröffentlichungsnummer: WO 2017/059835

(56) Entgegenhaltungen:
- EP-A1- 1 772 191
- DE-A1- 2 308 485
- DE-A1- 2 917 767
- US-A- 3 799 342
- US-A- 3 850 174
- US-A- 3 932 277
- US-A- 3 954 614

## Beschreibung

Die Erfindung betrifft einen Phasenseparator, der einen speziell hergerichteten Stopfen für ein Gefäß, vorzugsweise ein Zentifugationsgefäß, und ein Aufnahmebehältnis aufweist, und ein Verfahren zur Separierung eines flüssigen Überstands mittels des Phasenseparators.

Nicht nur diagnostische Tests sondern auch die Bereitstellung von Blutplasma im Sinne eines autologen Transplantats erfordern bei der Abtrennung von Blutplasma aus Vollblut weitestgehend steriles Arbeiten. Die Trennung von Vollblut in zelluläre Bestandteile und flüssige Bestandteile erfolgt durch Absetzung der dichteren Anteile aufgrund der Schwerkraft, durch andere technische Methoden und routinemäßig mittels Zentrifugation. Herkömmliche Verfahren weisen dabei einige Nachteile auf, die zu vermeiden Aufgabe der vorliegenden Erfindung ist.

Zu rein analytischen Zwecken in der Labormedizin läuft das Standardverfahren so ab, dass Vollblut mittels einer medizinischen Kolbenspritze in ein Zentrifugenröhrchen überführt wird, das mit einer Verschlusskappe versehen ist, indem die Verschlusskappe mit einer der das Vollblut enthaltenden medizinischen Spritze aufgesetzten Kanüle an einer dafür geeignet ausgebildeten Stelle durchstoßen wird, um dann unter Betätigung des Spritzenkolbens das Vollblut in das Zentrifugenröhrchen zu überführen, wobei bedarfsweise gerinnungshemmende Beschichtungen zum Einsatz kommen können. Nach dem anschließenden Zentrifugationsschritt und Ausbildung einer Phase mit zellulären Blutbestandteilen am Boden des Zentrifugenröhrchens und einem Überstand aus flüssigem Blutplasma wird die Verschlusskappe erneut mit einer Kanüle, die einer medizinischen Kolbenspritze aufgesetzt ist, durchstochen und der Überstand mit dem Blutplasma vorsichtig abgesaugt. Problematisch ist dabei, dass der Absaugvorgang mittels einer Kanüle lediglich in ausreichendem Abstand zur Phasengrenze trennscharf erfolgen kann, da mit zunehmender Verringerung des Abstands ansaugbedingte Verwirbelungen zu einer unerwünschten Vermischung der Phasen führen und häufig einen Verzicht auf den phasengrenznahen Bereich des Überstandes erfordern, der aufgrund der Dichteverteilung die für viele Anwendungen wertvolle plättchenreiche Fraktion enthält.

Zur Vereinfachung der Phasentrennung sind unterschiedliche Verfahren und Vorrichtungen im Stand der Technik beschrieben. Die EP 1 014 088 B1 offenbart ein Verfahren zum Separieren einer Flüssigkeitsprobe in eine Phase höherer Dichte und eine Phase geringerer Dichte bei dem ein Separatorelement, das im Ruhezustand durch eine elastomere Membran gegen die Innenwand eines Zentrifugenröhrchens abgedichtet ist und so ausgeführt ist, dass sich die elastomere Membran unter Einwirkung der Zentrifugalkräfte von der Innenwand des Zentrifugenröhrchens löst mit der Folge, dass das Separatorelement sich seiner Dichte entsprechend zwischen zwei Phasen anordnet und sich im Ruhezustand nach der Zentrifugation wieder dichtend an die Innenwand des Zentrifugenröhrchen schmiegt. Die obere Phase kann dann wie beim Standardverfahren gewonnen werden, jedoch ohne die Gefahr der unerwünschten Verwirbelungen während der Abnahme des Überstands. Gleichwohl können die unerwünschten Verwirbelungen bei diesem Verfahren bereits während der Zentrifugation stattfinden, da sich das Separatorelement während der Zentrifugation durch das zu trennende flüssige Gemisch bewegt und dabei unkontrollierbare Effekte auf die Schichtung von Molekülen und subzellulären Strukturenbestandteilen ausübt. Nachteilig ist bei diesem Verfahren außerdem, dass das Zentrifugenröhrchen während des gesamten Verfahrens mit einer aufgesetzten Verschlusskappe versehen ist, die zweimal durchstochen werden muss, nämlich einmal bei der Einbringung von Vollblut in das Zentrifugenröhrchen und erneut, um den separierten Überstand nach der Zentrifugation absaugen zu können. Jedes Durchstechen birgt jedoch die Gefahr unerwünschter Kontamination.

Die DE 195 13 453 C2 offenbart eine ähnliche Lösung wie die EP 1 014 088 B1 mit dem Unterschied, dass das Separatorelement hier schlichter ausgestaltet ist und nur einen der Wandung des Zentrifugenröhrchens dichtend anliegenden Zustand erlaubt. Auch hier wandert das Separatorelement während der Zentrifugation. Es weist einen Durchlass auf, durch den die zu gewinnende Phase hindurchtritt, wenn sich das Separatorelement in dem Zentrifugenröhrchen nach unten bewegt. Die Bewegung des Separatorelementes stoppt, wenn es auf einen vom Boden in das Zentrifugenröhrchen ragenden Dorn aufsitzt, der dann den Durchlass durchdringt. Nachteilig ist hierbei wiederum, dass die Entnahme des separierten Überstandes ein erneutes Durchstechen der dem Zentrifugenröhrchen aufgesetzten Verschlusskappe erforderlich macht. Weiterhin nachteilig ist die durch die Form des Dorns festgelegte Endposition des Separatorelementes, die keine Anpassung an unterschiedliche zu trennende flüssige Gemische erlaubt, deren Phasengrenzen entsprechend unterschiedliche Positionen aufweisen können. Das offenbarte Verfahren ist zudem nicht auf die Gewinnung autologer Transplantate ausgelegt, da der Einsatz von Gleitgelen für das Separatorelement gelehrt wird, deren Anwesenheit sich verbietet, soweit der zu gewinnende Überstand einem Patienten reinfundiert werden soll.

Die US 4 050 451, die DE 29 29 216 A1 sowie die EP 0 073 551 A2 offenbaren sämtlich den Einsatz thixotroper Gele, die sich aufgrund ihrer Dichten nach Zentrifugation zwischen zwei zu trennenden Phasen anordnen. Ein Kontakt des zu trennenden Überstandes mit Gel, gleich welcher Zusammensetzung, ist jedoch unerwünscht, wenn nicht nur zu rein laboranalytischen Zwecken separiert werden soll.

Die EP 0 627 261 B1, die EP 0 744 026 B1 sowie die Offenlegungsschrift DE 1 806 196 offenbaren sämtlich so genannte Trennungsschwimmer, die sich aufgrund ihrer Dichte während des Zentrifugationsvorgangs zwischen den zu trennenden Phasen positionieren. Das Problem der Entnahme der separierten Phase wird hier jedoch nicht adressiert und es verbleibt bei dem bekannten Durchstechen einer Verschlusskappe sowohl bei Einbringung des zu trennenden flüssigen Gemischs als auch bei der Entnahme der separierten Phase mit den oben genannten Nachteilen.

DE 23 08 485 A1 offenbart eine Vorrichtung zum Trennen eines flüssigen Gemisches in leichte und schwere Phasen, insbesondere zum Trennen von Blut in seine leichte, aus Plasma oder Serum bestehende Phase und in seine schwere, aus Zellen bestehende Phase vorzugsweise durch Fliehkraft, die gekennzeichnet ist durch einen Behälter zur Aufnahme der Flüssigkeit mit mindestens einem offenen Ende, das durch einen Stopfen verschließbar ist, einen verschiebbar in dem Behälter angeordneten Kolben, dessen Dichte relativ größer ist als die schwere Phase und der an seiner Außenfläche Dichtungsmittel zum dichten Zusammenwirken mit der Innenwand des Behälters aufweist, Mittel zur Bewegung des Kolbens in dem Behälter, um einen beträchtlichen Teil der leichten Phase von der schweren Phase zu trennen, auf Druck ansprechende Ventilmittel im Kolben, die normalerweise geschlossen sind, jedoch bei einem vorbestimmten Druck, wenn sich der Kolben verschiebt, öffnen und sich automatisch schließen, wenn der Kolben zum Stillstand kommt, und einen Anschlag im Behälter für den Kolben am Ende seines die leichte von der schweren Phase trennenden Weges. Nachteilig ist hier, dass die Entnahme der abgetrennten leichten Phase nicht näher adressiert wird. Der Behälter wird einerseits zum Befüllen mit dem zu trennenden Gemisch geöffnet oder der Verschlussstopfen mittels einer Kanüle durchstoßen und andererseits für die Entnahme ein zweites Mal geöffnet oder der Verschlussstopfen wird erneut durchstoßen. Das Kontaminationsrisiko wird dabei mit jedem zusätzlichen Öffnen oder Durchstechen gesteigert.

US 3 799 342 A offenbart ein Verfahren zur Trennung von Serum oder Plasma von im Blut befindlichen Formbestandteilen, das Schritte umfasst zur Fällung der Formbestandteile auf den Boden eines Behältnisses und das Einführen eines Stopfens in das Behältnis, der normalerweise geschlossene Ventilelemente aufweist, die sich öffnen können, wenn der Druck in dem Behältnis größer als der atmospärische Druck ist. Ein Griffstück zur Handhabung des Stopfens fungiert als Behälter für die Sammlung und Entfernung von Serum oder Plasma. In einer zweiten Ausführungsform ist das Griffstück lösbar mit dem Stopfen verbunden, so dass der Stopfen in dem Behältnis verbleiben kann, um die Trennung zwischen dem Serum oder Plasma und den Formbestandteilen des Blutes zu erhalten. In einer dritten Ausführungsform wird der Stopfen mit einem Behälter zum Sammeln und Lagern des Serums oder Plasmas bereitgestellt. Ein ähnlicher Stopfen innerhalb des Behältnisses wird für einen zusätzlichen Filtrationsschritt genutzt. Von Nachteil ist hier, dass das Griffstück an seinem oberen Ende Ventilelemente aufweist, damit bei der Aufnahme des abgetrennten Serums oder Plasmas Luft aus dem Behälter des Griffstücks entweichen kann. Ähnlich wie die Befüllung des für den Trennvorgang vorgesehenen Behältnisses stellt auch die Öffnung der Ventilelemente des Sammelbehälters am Griffstück eine zusätzliche Kontaminationsgefahr dar.

US 3 850 174 A offenbart eine Anordnung zur Aufnahme von in eine leichte flüssige Phase aus Serum oder Plasma und in eine schwere Phase zu trennendem Blut. Die Anordnung umfasst ein selbstdichtendes, durchstoßbares, elastomeres Stopfen-/ Kolbenelement, das sowohl als gleitfähiger Kolben als auch als Verschluss zur Erhaltung eines Vakuums in einem einem evakuierten Blutsammeigefäß dienen kann. Ein Mittel, geeignet das Kolbenelement nach unten zu drücken, wird bereitgestellt; an dem Mittel ist ein spitzes, röhrenförmiges Element angeordnet, das dazu geeignet ist, den Kolben zu durchstoßen und so einen Durchgang zu schaffen für die Durchleitung der abgetrennten, leichten flüssigen Phase von der einen Seite des Stopfens / Kolbens auf dessen andere Seite. Auch hier wird das Durchstoßen des die Phasen trennenden Stopfen- / Kolbenelements gelehrt. Sowohl für die Befüllung mit dem aufzutrennenden Blut als auch die Entnahme der leichten flüssigen Phase erfordert jeweils ein Öffnen des Blutsammelgefäßes mit entsprechend zweimaliger Kontaminationsgefahr.

Es stellt sich daher die Aufgabe, die Anzahl der erforderlichen Durchstiche zu reduzieren.

Die Nachteile des Standes der Technik werden mittels des Verfahrens nach Anspruch 7 weitestgehend überwunden, bei dem ein Phasenseparator gemäß Anspruch 1 zum Einsatz kommt, der einen speziell hergerichteten Stopfen und ein Aufnahmebehältnis gemäß Anspruch 1 aufweist. Vorteilhafte Ausführungsformen ergeben sich aus den jeweiligen Unteransprüchen.

Der Stopfen ist dabei so ausgeführt, dass er zum Verschließen eines eine einendseitige Öffnung und eine Innenwandung aufweisenden Gefäßes durch Presspassung mit der Innenwandung geeignet ist, und weist dabei einen Durchlass auf, der von einer Membran verschlossen ist. Material und Stärke der Membran sind dabei so gewählt, dass die Membran mittels einer Kanüle durchstoßen werden kann und dadurch bei händisch erzeugtem Druck für Flüssigkeiten durchlässig wird. Vorzugsweise ist die Membran elastisch, beispielsweise dadurch, dass sie aus Gummi oder einem anderen elastomeren Material gefertigt ist. Gleichwohl ist es unerwünscht, dass die Elastizität des Materials und die gewählte Stärke dazu führen, dass trotz händisch erzeugten Drucks keine Flüssigkeit mehr durch den erzeugten Durchstich dringen kann. Besonders bevorzugt ist es, wenn der Durchstich sich aufgrund der Materialelastizität der Membran nach Entfernen der für den Durchstich verwendeten Kanüle wieder schließt und bei händisch erzeugtem Druck gegen eine Flüssigkeit sich wieder so weit öffnet, dass die Flüssigkeit hindurch treten kann, so dass Kontaminationen von der Seite der Membran, von der aus der Durchstich erfolgte, weitestgehend ausgeschlossen sind. Auf einer Seite der Membran weist der Stopfen einen stutzenartigen Ansatz auf, der vorzugsweise röhrenförmig ausgestaltet ist, wobei das eine offene Ende des Ansatzes vorzugsweise der Membran aufliegt, während das andere offene Ende des Ansatzes bevorzugt über den restlichen Teil des Stopfens hinaussteht, um eine einfache Kopplungsmöglichkeit für ein Aufnahmebehältnis bereit zu stellen. Selbstverständlich sind dabei auch andere Ausführungsformen möglich, bei denen der Ansatz in den restlichen Teil des Stopfens integriert und beispielsweise von einer umlaufenden Vertiefung umgeben ist. In einer einfachen Ausführungsform kann der Ansatz auch als entsprechend ausgeformte Aussparung in dem Stopfen ausgestaltet sein. Bei einer solchen Ausführungsform ist eine kostengünstige Herstellung des Stopfens als einstückiges Element möglich. Das Durchstechen der Membran ist dann von der Seite des Ansatzes her vorgesehen.

Für die Presspassung ist es von Vorteil, wenn der Stopfen eine oder mehrere Dichtlippen aufweist, die vorzugsweise elastisch und umlaufend sind. Für die Möglichkeit einer verkantungsfreien Positionierung des Stopfens in dem die Innenwandung für die Presspassung aufweisenden zu verschließenden Gefäß ist es vorteilhaft, wenigstens zwei umflaufende Dichtlippen vorzusehen, von denen eine den oberen Teil des Stopfens umgibt und die andere den unteren Teil des Stopfens. Bei einem Gefäß mit rundem Querschnitt, wie einem Zentrifugationsröhrchen, ist beispielsweise ein zylindrischen Stopfen von Vorteil, der eine umlaufende elastische Dichtlippe jeweils an der oberen und unteren Kante zwischen Mantel- und Kreisfläche aufweist und dessen Höhe nicht kleiner als der Durchmesser des Gefäßquerschnitts ist.

Für eine einfache Handhabung des Stopfens, insbesondere bei dem Durchstechen der Membran, ist es von Vorteil, wenn eine Mittelachse des Stopfens durch den gesamten Durchlass verläuft, bei einem zylindrischen oder kegelstumpfförmigen Stopfen der Durchlass also in beiden Kreisflächen zentriert angeordnet ist. Je nach Form der Innenwand des Gefäßes sind aber auch andere, beispielsweise würfelförmige Stopfenformen möglich.

Grundsätzlich ist der stutzenartige Ansatz des Stopfens so ausgebildet, dass er mit einem entsprechend ausgebildeten Aufnahmegefäß koppelbar ist. Stutzenartig bedeutet hier, dass der Ansatz wenigstens eine Ausnehmung aufweist, in die der Durchlass des Stopfens übergeht. Von Vorteil ist es, wenn der Ansatz mit dem Auslass eines als Kolbenspritze ausgebildeten Aufnahmebehältnisses koppelbar ist, da im medizinischen Bereich Kolbenspritzen weit verbreitet und unproblematisch verfügbar sind. Im Normalfall weisen solche medizinischen Kolbenspritzen einen Auslass am Ende eines Stutzens auf, der beispielsweise bei der erwünschten Kopplung so in den stutzenförmigen Ansatz gesteckt werden kann, dass die Verbindung nach außen flüssigkeitsdicht ist. Zur Vermeidung eines versehentlichen Lösens einer solchen Kopplung können auch Schraubgewinde Verwendung finden, bei denen vorzugsweise bereits nach einer halben Drehung der zu verbindenden Teile ein Anschlag erreicht wird und ein versehentliches Lösen der Verbindung dadurch weitestgehend ausgeschlossen ist. Auch andere Verbindungen sind möglich, wie beispielsweise Bajonettverschlüsse. Möglich ist auch die Verbindung des Ansatzes mit dem Aufnahmegefäß über einen Schlauch.

Um unterschiedliche Aufnahmebehältnisse an einen Stopfen koppeln zu können, ist es vorteilhaft, wenn der Ansatz als separates Bauteil ausgeführt ist, so dass die Anpassung des Stopfens produktionstechnisch durch entsprechenden Austausch des Ansatzes auf kostengünstige Weise verwirklicht werden kann. Ein als separates Bauteil ausgeführter Ansatz erleichtert auch einen Materialmix, bei dem der Ansatz beispielsweise aus steifem Kunststoff gefertigt ist, während der übrige Stopfen aus elastischem Material besteht.

Eine Ausführungsform des Ansatzes, bei dem sich der Ansatz in Richtung der Membran verjüngt, ist von Vorteil, da so eine Führung der Kanüle für einen erwünschten zentrierten Durchstich erreicht werden kann.

Zwar ist es möglich, den Stopfen im Verhältnis zu dem das zu trennende flüssige Gemisch aufweisende Gefäß so zu dimensionieren, dass die Presspassung auch bei moderaten Zentrifugationsgeschwindigkeiten eine Verschiebung des Stopfens zuverlässig verhindert, jedoch ist es ebenso möglich, die Dichte des Stopfens so zu wählen, dass sie geringer als die des zu gewinnenden Überstands aus dem zu trennenden flüssigen Gemisch ist, also beispielsweise geringer als die Dichte menschlichen Blutplasmas. Dadurch kann selbst bei unzureichender Presspassung im Verhältnis zur Zentrifugationsgeschwindigkeit verhindert werden, dass der Stopfen sich in die Flüssigkeit hinein bewegt.

Mit dem Stopfen, einem bevorzugt als Kolbenspritze ausgestaltetem Aufnahembehältnis und einem Gefäß, das eine einendseitige Öffnung, eine Innenwandung und einen anderendseitigen Boden aufweist, lässt sich ein Phasenseparator bereit stellen, bei dem der Stopfen mit der Innenwandung eine Presspassung bildet und die Öffnung verschließt, wobei der Durchlass des Stopfens parallel zu der Innenwandung verläuft, der Stopfen entlang der Innenwandung verschieblich und die Membran zwischen dem Boden und dem Ansatz des Stopfens angeordnet ist. Das Gefäß des Phasenseparators kann dann mit einem zu trennenden flüssigen Gemisch befüllt werden, indem die Membran des Stopfens mittels einer Kanüle durchstoßen wird, durch die dann das flüssige Gemisch in das Gefäß fließen kann. Nach dem Befüllen kann die Kanüle dann entfernt werden, wobei die Membran des Stopfens dann einen bei händisch erzeugbarem Druck für Flüssigkeiten durchlässigen Zustand aufweist.

Um seine Funktion zuverlässig erfüllen zu können, weist der Phasenseparator bevorzugt ein Gefäß auf, dass parallel zu der Mittelachse des Stopfens verlaufende Abschnitte der Innenwandung aufweist, die einander parallel gegenüberliegend angeordnet sind und dem Stopfen auf jeweils gleichen, zur Mittelachse orthogonalen Ebenen anliegen, so dass der Stopfen achsstabil parallelverschieblich zu diesen Abschnitten der Innenwandung in dem Gefäß angeordnet ist. So ist sichergestellt, dass der Stopfen während des Verschiebens in Richtung des Gefäßbodens nicht verkantet, was zu unerwünschten Verwirbelungen nach Phasentrennung führen könnte. Der Stopfen kann dabei ein Gefäß mit einem röhrenförmigen Bereich verschließen, wenn er eine zylindrische oder kegelstumpfförmige Form aufweist, wobei der Zylinder oder Kegelstumpf an seiner Ober- und Unterkante jeweils eine ringförmige Verbreiterung aufweist, beispielsweise in Form einer oberen und einer unteren Dichtlippe. Zum Schutz gegen Verkantungen reicht es aber auch aus, wenn die Verbreiterungen nicht umlaufend sind, wie bei einer Dichtlippe, sondern an einigen Stellen unterbrochen, solange zwischen den Verbreiterungen an Ober- und Unterkante des Zylinders oder Kegelstumpfes wenigstens eine umlaufende Dichtlippe angeordnet ist.

Für Anwendungen zur Gewinnung von menschlichem Blutplasma im Sinne eines autologen Transplantats ist es vorteilhaft, wenn das Gefäß des Phasenseparators evakuiert ist, der von dem Stopfen verschlossene Raum des Gefäßes also einen Unterdruck gegenüber dem Umgebungsdruck aufweist. So ist es möglich, einem Patienten durch Punktion eines Blutgefäßes Blut abzunehmen, wobei der Ort der Punktion mit der Kanüle zum Durchstechen der Membran des Stopfens über einen Schlauch verbunden ist, so dass nach Durchstechen der Membran das Blut des Patienten in das Gefäß des Phasenseparators gesaugt wird.

Von Vorteil ist es, wenn der Phasenseparator so ausgeführt ist, dass der Ansatz des Stopfens mit dem Auslass einer Kolbenspritze koppelbar ist, wobei die Kolbenspritze als Handhabe für die Verschiebung des Stopfens in das Gefäß des Phasenseparators ganz oder teilweise einbringbar ist. Im Falle eines röhrenförmigen Zentrifugationsgefäßes sollte also der Außendurchmesser einer ankoppelbaren medizinischen Kolbenspritze kleiner sein als der Innendurchmesser des Zentrifugationsgefäßes, so dass mittels der angekoppelten Kolbenspritze der Stopfen händisch in Richtung des Gefäßbodens bewegt werden kann.

Für Gefäße des Phasenseparators, die als Zentrifugationsgefäße ausgeführt sind, ist es vorteilhaft für Transport- und Lagerungszwecke, wenn der Öffnung des Zentrifugationsgefäßes eine Schutzkappe aufgesetzt ist, die mittels der Kanüle, die auch zum Durchstoßen der Membran des Stopfens verwendet wird, durchstoßbar ist. Anders als bei der Membran des Stopfens ist es dabei nicht erforderlich, dass die Schutzkappe durch das Durchstoßen in einen flüssigkeitsdurchlässigen Zustand versetzt wird. Sie kann also beispielsweise an der für das Durchstoßen vorgesehenen Stelle eine Gummilage aufweisen, die so dick ist, dass die durch das Durchstoßen verursachte Ausnehmung aufgrund der Materialelastizität wieder verschlossen wird und bei händisch erzeugbarem Druck keinen Durchtritt von Flüssigkeit erlaubt. Gleichwohl ist es vorteilhaft, wenn der Stopfen so dicht unter der Schutzkappe angeordnet ist, dass die Schutzkappe und die Membran mit ein und derselben Kanüle in einer Bewegung durchstochen werden können. Zum Ankoppeln des Aufnahmegefäßes an den Phasenseparator ist die Schutzkappe dann zu entfernen. Phasenseparatoren mit Schutzkappe können auch so vorgefertigt werden, dass die Membran bereits in einen bei händisch erzeugbarem Druck für Flüssigkeiten durchlässigen Zustand versetzt wurde, wobei es dann vorteilhaft ist, wenn die Membran sich bei Abwesenheit von Druck dichtend verschließt.

Um eine genaue Abstimmung des Aufnahmebehältnisses auf den Ansatz des Phasenseparators zu gewährleisten, ist es von Vorteil, den Phasenseparator mit dem Aufnahmebehältnis möglichst bereits vorkonfektioniert bereit zu stellen, wobei das Aufnahmebehältnis lösbar und flüssigkeitsdicht mit dem Ansatz verbunden ist. Im Falle eines als Zentrifugationsgefäß ausgeführten Gefäßes des Phasenseparators kann das Aufnahmebehältnis dann zunächst vor Durchstechen der Membran und Befüllen des Zentrifugationsgefäßes abgekoppelt werden, um nach erfolgter Phasentrennung wieder angekoppelt zu werden. Dabei muss die Kopplung zwischen dem Aufnahmebehältnis und dem Ansatz des Stopfens nicht zwingend direkt erfolgen. Sie kann beispielsweise auch vermittels eines Schlauchs erfolgen, wobei dann die vorteilhafte Möglichkeit, das Aufnahmebehältnis als Handhabe für das Verschieben des Stopfens zu verwenden in den meisten Fällen, je nach Steifigkeit und Länge des Schlauchs, entfällt. Stattdessen kann eine andere Handhabe verwendet werden, beispielsweise ein Stab, ein Schieber, ein Stopfinstrument oder dergleichen.

Mit dem Phasenseparator, der ein an den Ansatz des Stopfens mittelbar oder unmittelbar ankoppelbares Aufnahmebehältnis umfasst, ist es möglich, ein Verfahren zur Phasentrennung eines flüssigen Gemischs und nachfolgender Separierung eines flüssigen Überstands durchzuführen, bei dem unter weitestgehend sterilen Bedingungen gearbeitet werden kann, da die Membran des Stopfens lediglich einmal durchstoßen werden muss. In einem ersten Schritt wird dabei die Membran des Stopfens mit einer Kanüle durchstoßen, wobei das zu trennende flüssige Gemisch durch die Kanüle in das Gefäß des Phasenseparators fließt. Das zu trennende flüssige Gemisch kann dabei direkt vom Patienten abgenommenes Blut sein oder ein anderes, frei wählbares flüssiges Gemisch aus einem Reservoir, das vorzugsweise mit der Kanüle bereits verbunden ist. In einem zweiten Schritt wird die Kanüle aus der Membran entfernt. Die Membran befindet sich danach in einem bei händisch erzeugbarem Druck für Flüssigkeiten durchlässigen Zustand und ist ohne Druckausübung vorzugsweise aufgrund der Materialelastizität der Membran oder aufgrund anderer Eigenschaften oder ventilartiger Ausgestaltungen flüssigkeitsdicht. In einem dritten Schritt wird die Ausbildung eines flüssigen Überstands aus dem zu trennenden flüssigen Gemisch in dem Gefäß des Phasenseparators entweder aktiv oder passiv herbeigeführt, wobei unter einem passiven Herbeiführen der Phasentrennung hier das Einwirken der natürlichen Schwerkraft im Zeitverlauf verstanden wird. Bevorzugt wird die aktive Herbeiführung der Ausbildung des zu separierenden flüssigen Überstands, vorteilhafterweise mittels Zentrifugation. In einem vierten Schritt wird das Aufnahmebehältnis mit dem Ansatz des Stopfens verbunden und in einem fünften Schritt wird, ohne dass es noch einmal eines Durchstoßens der Membran bedarf, der Stopfen in Richtung des Bodens verschoben bis der zu separierende Überstand durch den Durchlass gedrungen ist und so in das Aufnahmebehältnis gelangt.

Nach Aufnahme des Überstandes in dem Aufnahmebehältnis kann das Aufnahmebehältnis in einem sechsten Schritt von dem Phasenseparator abgekoppelt werden, so dass der Überstand in dem Aufnahmebehältnis zur weiteren Verwendung verfügbar ist.

Eine vorteilhafte Ausführung des Verfahrens wird dadurch ermöglicht, dass das Aufnahmebehältnis in das Gefäß des Phasenseparators ganz oder teilweise einbringbar ist und so als Handhabe für die Verschiebung des Stopfens verwendet werden kann. Dabei kann das Aufnahmebehältnis einen Außendurchmesser aufweisen, der kleiner als der Innendurchmesser eines Gefäßes mit röhrenförmigem Einlass ist und so falls gewünscht ganz in das Gefäß eingebracht werden. Ebenso ist es möglich, dass das Aufnahmebehältnis einen Stutzen aufweist, dessen Abmessungen ein Einbringen des Stutzens in die Öffnung des Gefäßes erlauben.

Bevorzugt wird das Verfahren durchgeführt mit einem als Kolbenspritze ausgestalteten Aufnahmebehältnis, das beim Verschieben des Stopfens ganz oder teilweise in das Gefäß eingebracht wird, wobei der Kolben der Kolbenspritze durch den separierten flüssigen Überstand aus dem Spritzenkörper herausgedrückt wird.

Selbstverständlich sind Phasentrennungen möglich, die mehr als zwei Phasen aus einem flüssigen Gemisch abtrennen. Um beispielsweise die mittlere von drei Phasen zu separieren, werden die oben beschriebenen Schritte drei bis sechs nach Schritt sechs wiederholt, wobei in dem vierten Schritt jeweils ein neues, noch unbenutztes Aufnahmebehältnis verwendet wird. Dadurch kann zunächst die oberste Phase separiert werden und danach die folgende Phase. Analog können diese Verfahrensschritte entsprechend der Anzahl der Phasen wiederholt werden bis alle zu separierenden Phasen getrennt voneinander in jeweils verschiedenen Aufnahmebehältnissen verfügbar sind.

Nachfolgend werden der Phasenseparator (14) und das Verfahren zur Phasentrennung eines flüssigen Gemischs (19) und nachfolgender Separierung eines flüssigen Überstands (20) anhand bevorzugter Ausführungsformen und der Figuren beispielhaft näher erläutert.

| | | | |
|---|---|---|---|
| | Legende: | 11 | Mittelachse |
| 1 | Stopfen | 12 | Auslass |
| 1a | Ebenen | 13 | Kolbenspritze |
| 2 | Öffnung | 14 | Phasenseparator |
| 3 | Innenwandung | 15 | Boden |
| 3a | Abschnitte der Innenwandung | 16 | verschlossener Raum |
| 4 | Gefäß | 17 | Spritzenkörper |
| 5 | Membran | 18 | Kolben |
| 6 | Durchlass | 19 | flüssiges Gemisch |
| 7 | Ansatz | 20 | flüssiger Überstand |
| 8 | Aufnahmebehältnis | 21 | Schutzkappe |
| 9 | Kanüle | 22 | Anordnung |
| 10 | Dichtlippe | | |

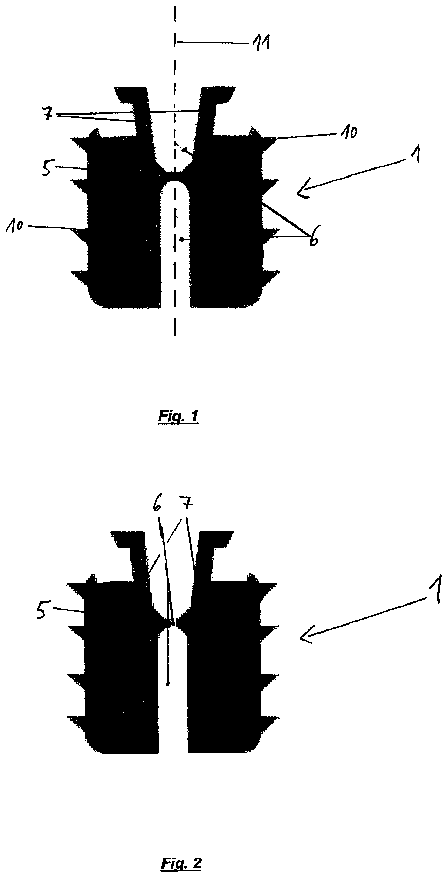

**Fig. 1** zeigt den Stopfen (1) im Querschnitt mit dem von der Membran (5) verschlossenen Durchlass (6), dem Ansatz (7), den Dichtlippen (10) und der Mittelachse (11), die durch den gesamten Durchlass (6) verläuft. Die Dichtlippen (10) sind hier unten angeschrägt, um eine Abwärtsbewegung innerhalb eines Gefäßes gegenüber einer Aufwärtsbewegung zu erleichtern.
**Fig. 2** zeigt den Stopfen (1) im Querschnitt mit dem Durchlass (6) und mit der durchstoßenen, bei händisch erzeugbarem Druck für Flüssigkeiten durchlässigen Membran (5), wobei die dieser Zustand der Membran (5) hier durch eine Lücke in der Membranmitte angedeutet wird, was jedoch nicht zwingend den tatsächlichen Gegebenheiten entsprechen muss, da sich die durchstoßene Membran wünschenswerterweise erst aufgrund ihrer Elastizität und aufgrund des händisch erzeugten Flüssigkeitsdrucks öffnet und ohne den erzeugten Druck wieder flüssigkeitsdicht verschließt. Nachfolgend ist dieser Membranzustand weiterhin durch eine Lücke in der Membran (5) dargestellt.

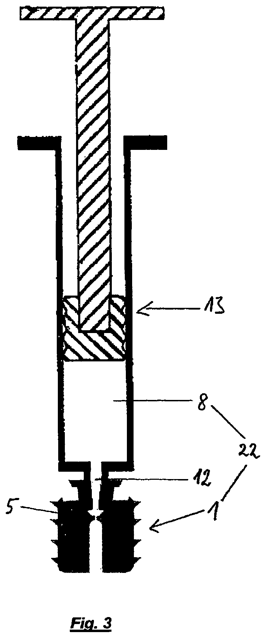

**Fig. 3** zeigt einen Querschnitt einer Anordnung (22) aus dem Stopfen (1) mit durchstoßener Membran (5) und dem Aufnahmebehältnis (8), das hier als Kolbenspritze (13) mit Auslass (12) ausgeführt ist.

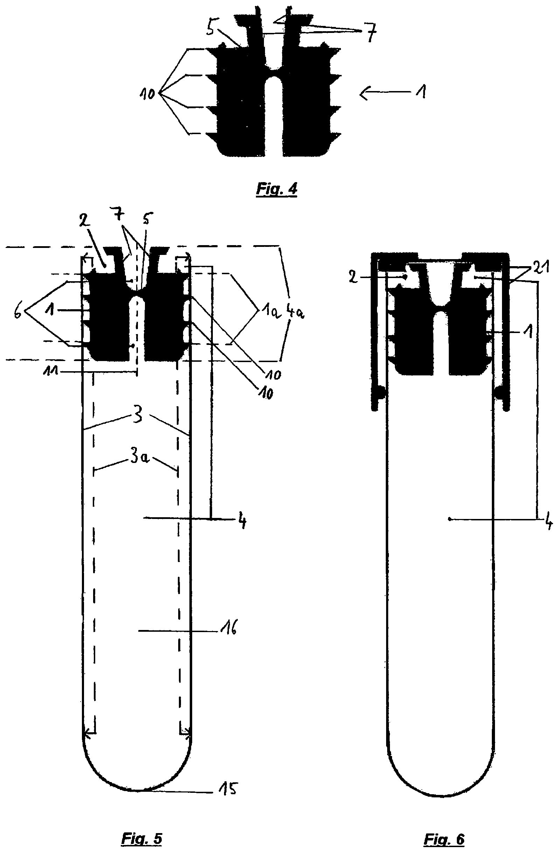

**Fig. 4** zeigt den Stopfen (1) im Querschnitt mit den hier umlaufenden Dichtlippen (10) und dem als separates Bauteil ausgeführten Ansatz (7), der sich in Richtung der Membran (5) verjüngt.
**Fig. 5** zeigt einen Querschnitt des Phasenseparators (14) nach Anspruch 1 mit dem Stopfen (1), der die einendseitige Öffnung (2) des Gefäßes (4) verschließt, das hier als Zentrifugenröhrchen ausgebildet ist, indem er mit der Innenwandung (3) des Gefäßes (4) eine hier von den Dichtlippen (10) vermittelte Presspassung bildet, wobei der Stopfen (1) hier so angeordnet ist, dass dessen von der Membran (5) verschlossener Durchlass (6) zwei zu der Innenwandung (3) des Gefäßes (4) orthogonal verlaufende Ebenen (4a) verbindet, wobei der Stopfen (1) entlang der Innenwandung (3) achsparallel verschieblich zu einem Abschnitt (3a) der Innenwandung (3) angeordnet ist, der parallel zu der Mittelachse (11) des Stopfens (1) verläuft, so dass der Abschnitt (3a) dem wenigstens zum Teil in dem Gefäß (4) angeordneten Stopfen (1) auf zu der Mittelachse (11) orthogonalen Ebenen (1a) mit jeweils gleichem Druck anliegt, und die Membran (5) zwischen dem Boden (15) des Gefäßes (4) und dem Ansatz (7) des Stopfens (1) angeordnet ist. Der von dem Stopfen (1) verschlossene Raum (16) des Gefäßes zwischen dem Boden (15) und der Membran (5) weist gegenüber dem Umgebungsdruck einen Unterdruck auf. Der Ansatz (7) des Stopfens (1) ist mit dem Auslass (12) der Kolbenspritze (13) koppelbar, die als Handhabe für die Verschiebung des Stopfens (1) ganz oder teilweise in das Gefäß (4) einbringbar ist.

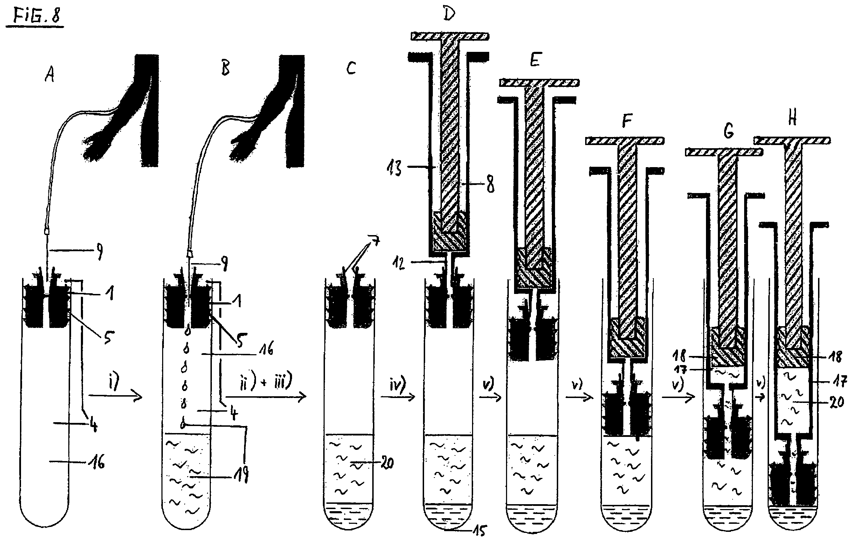

**Fig. 6** zeigt den Phasenseparator (14) im Querschnitt mit einer der durch den Stopfen (1) verschlossenen Öffnung (2) des Gefäßes (4) aufgesetzten Schutzkappe (21), die mittels einer Kanüle **(****Fig. 8****,** (9)) durchstoßbar ist. Die Schutzkappe (21) schützt den Stopfen (1) vor Beschädigungen und unerwünschtem Herauswandern bei Konfektionierung und Transport des Phasenseparators (14). Sie (21) kann vorteilhafterweise lösbar mit dem Stopfen (1) verbunden sein und so ein unbeabsichtigtes Verschieben des Stopfens (1) in Richtung des Bodens (15), beispielsweise während eines Zentrifugationsschrittes, verhindern. Erst nach Lösen der Verbindung der Schutzkappe (21) mit dem Stopfen (1) kann der Stopfen (1) dann in Richtung des Bodens (15) verschoben werden. Die Verbindung kann beispielsweise mittels versteifter Laschen des Stopfens (1), die in den Innenrand der Schutzkappe (21) greifen, hergestellt sein.

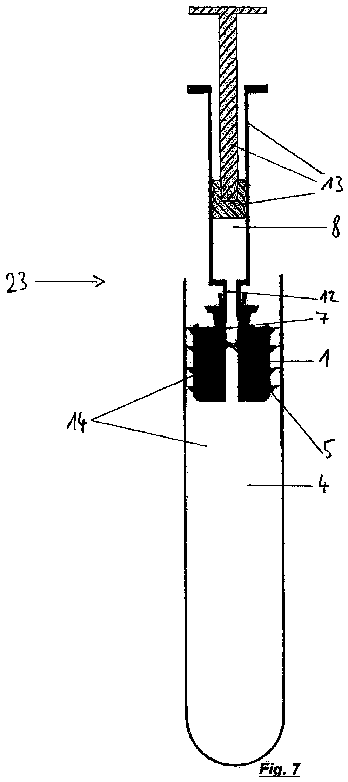

**Fig. 7** zeigt im Querschnitt den Phasenseparator (14) mit dem Aufnahmebehältnis (8), wobei die Membarn (5) des Phasenseparators (14) bereits durchstoßen und bei händisch erzeugtem Druck für Flüssigkeiten durchlässig ist und das Aufnahmebehältnis (8), hier in Form einer Kolbenspritze (13) mit dem Auslass (12), lösbar und flüssigkeitsdicht mit dem Ansatz (7) des Stopfens (1) verbunden ist. Der Ansatz (7) ist hier als separates Bauteil ausgeführt, vorzugsweise als sog. Luer-Lock-Ansatz. In dieser Ausführungsform ermöglicht es der Phasenseparator auch, das Aufnahmegefäß (8) zunächst zum Einbringen des zu trennenden flüssigen Gemischs in das Gefäß (4) zu verwenden und nach erfolgter Phasentrennung zur Aufnahme des flüssigen Überstands.
**Fig. 8** skizziert das Verfahren nach Anspruch 7, wobei das Gefäß (4) des Phasenseparators (14) ein Zentrifugenröhrchen ist und die verwendeten Komponenten im Querschnitt gezeigt sind. Für die hier gezeigte Gewinnung von Plasma aus Frischblut wird eine Patientenvene über einen Schlauch mit der Kanüle (9) verbunden (A), mit der dann die Membran (5) des Stopfens (1) durchstoßen wird, wobei das Blut (19) durch die Kanüle (9) in das Gefäß (4) fließt, was durch den Unterdruck innerhalb des Raumes (16) des Gefäßes (4) befördert wird (B), entsprechend Schritt i) des Verfahrens. In Schritt ii) wird nach Einbringen der gewünschten Blutmenge (19) in das Zentrifugenröhrchen (4) die Kanüle (9) aus der Membran (5) entfernt und in Schritt iii) wird, hier mittels Zentrifugation, der zu separierende flüssige Plasmaüberstand (20) ausgebildet (C). In Schritt iv) wird das Aufnahmebehältnis (8), das hier als Kolbenspritze (13) mit Auslass (12) ausgebildet ist, flüssigkeitsdicht an den als separates Bauteil ausgebildeten Ansatz (7) gekoppelt (D). In Schritt v) wird der Stopfen (1) mittels der Kolbenspritze (13) als Handhabe in Richtung des Bodens (15) des Gefäßes verschoben, bis das zu separierende Plasma (20) durch die Membran (5) des Stopfens in das Aufnahmebehältnis (8), hier also in die Kolbenspritze (13) gedrungen ist, wobei die Kolbenspritze (13) den Vorteil bietet, dass die Bewegung des Kolbens (18) nach und nach Volumen für die zu separierende Fraktion (20) in dem Spritzenkörper (17) freigibt (E, F, G, H). Das Aufnahmebehältnis (8) kann danach in einem Schritt vi) abgekoppelt werden und die Plasmafraktion (20) steht zur weiteren Verwendung bereit. Selbstverständlich können die Schritte iii) bis vi) einmal oder mehrmals wiederholt werden, wobei in Schritt iv) jeweils ein neues, noch unbenutztes Aufnahmebehältnis (8) verwendet werden sollte. So können übereinander geschichtete Fraktionen sukzessive voneinander separiert werden.

## Patentansprüche

1. Phasenseparator (14) für flüssige Gemische, insbesondere für menschliches Blut, mit einem einen Durchlass (6) aufweisenden Stopfen (1), einem eine einendseitige Öffnung (2), einen anderendseitigen Boden (15) und eine Innenwandung (3) aufweisenden Gefäß (4), dessen Öffnung (2) von dem Stopfen (1) durch Presspassung mit der Innenwandung (3) verschlossen ist, einem Aufnahmebehältnis (8) und einer den Durchlass (6) verschließenden Membran (5), die, bei manueller Verschiebung des Stopfens (1) entlang der Innenwand (3) in Richtung des Bodens (15) in eine in dem Gefäß (4) befindliche Flüssigkeit, für die Flüssigkeit durchlässig ist durch einen mittels einer zur Befüllung mit dem zu separierenden flüssigen Gemisch vorgesehenen Kanüle (9) erzeugten Durchstich,
**dadurch gekennzeichnet,**
**dass** der Durchlass (6) auf einer Seite der Membran (5) einen stutzenartigen Ansatz (7) zur Kopplung des Aufnahmebehältnisses (8) aufweist, die Membran zwischen dem Ansatz (7) und dem Boden (15) angeordnet ist und das Aufnahmebehältnis (8) als Handhabe für die manuelle Verschiebung des Stopfens (1) vorgesehen und als ganz oder teilweise in das Gefäß (4) einbringbare Kolbenspritze (13) mit einem an den Ansatz (7) koppelbarem Auslass (12), einem Kolben (18) und einem Spritzenkörper (17) ausgebildet ist, deren Kolben (18) von den Durchstich durchströmender Flüssigkeit aus dem Spritzenkörper (17) heraus bewegbar ist.

2. Phasenseparator (14) nach Anspruch 1,
**dadurch gekennzeichnet,**
**dass** die Membran (5) sich aufgrund ihrer Materialelastizität nach Entfernen der für den Durchstich verwendeten Kanüle (9) bei Ruhen des Stopfens (1) verschließt und bei manueller Verschiebung des Stopfens (1) entlang der Innenwand (3) in Richtung des Bodens (15) in die in dem Gefäß (4) befindliche Flüssigkeit vermittels des Aufnahmegefäßes (8) als Handhabe wieder so weit öffnet, dass die Flüssigkeit hindurch treten kann.

3. Phasenseparator (14) nach einem der vorhergehenden Ansprüche,
**dadurch gekennzeichnet,**
**dass** der Stopfen (1) eine geringere Dichte als menschliches Blutplasma aufweist.

4. Phasenseparator (14) nach einem der vorhergehenden Ansprüche,
**dadurch gekennzeichnet,**
**dass** der Stopfen (1) eine oder mehrere Dichtlippen (10) für die Presspassung aufweist.

5. Phasenseparator (14) nach einem der vorhergehenden Ansprüche,
**dadurch gekennzeichnet,**
**dass** das Gefäß (4) als Zentrifugationsgefäß mit einer der Öffnnung (2) aufgesetzten Schutzkappe (21) ausgeführt ist, wobei die Schutzkappe (21) mittels der Kanüle (9) durchstoßbar ist.

6. Phasenseparator (14) nach Anspruch 5,
**dadurch gekennzeichnet,**
**dass** die Schutzkappe (21) und der Stopfen (1) lösbar miteinander verbunden sind, wobei die Verschieblichkeit des Stopfens (1) erst nach Lösen der Verbindung gegeben ist.

7. Verfahren zur Phasentrennung eines flüssigen Gemischs (19) und nachfolgender Separierung eines flüssigen Überstands (20) mittels eines Phasenseparators (14) nach einem der Ansprüche 1 bis 6 und einer Kanüle (9),
**gekennzeichnet durch folgende Schritte:**
i) Durchstechen der Membran (5) mit der Kanüle (9), wobei das zu trennende flüssige Gemisch durch die Kanüle (9) in das Gefäß (4) fließt;
ii) Entfernen der Kanüle (9) aus der Membran (5);
iii) Ausbildung des zu separierenden Überstandes (20);
iv) Verbinden des Aufnahmebehältnisses (8) mit dem Ansatz (7), wobei das Aufnahmebehältnis (8) als Kolbenspritze (13) ausgestaltet ist;
v) Verschieben des Stopfens (1) in Richtung des Bodens (15) bis der zu separierende flüssige Überstand (20) durch die Membran (5) gedrungen ist, wobei das Aufnahmebehältnis (8) ganz oder teilweise in das Gefäß (4) eingebracht wird und der Kolben (18) der Kolbenspritze (13) durch den separierten flüssigen Überstand (20) aus dem Spritzenkörper (17) heraus gedrückt wird.

8. Verfahren nach Anspruch 7,
**dadurch gekennzeichnet,**
**dass** die Ausbildung des zu separierenden flüssigen Überstandes (20) in Schritt iii) mittels Zentrifugation erfolgt.

9. Verfahren nach einem der Ansprüche 7 oder 8,
das nach Schritt v) folgenden **Schritt umfasst:**
vi) Abkoppeln des Aufnahmebehältnisses (8).

10. Verfahren nach einem der Ansprüche 7 bis 9,
**dadurch gekennzeichnet,**
**dass** das Verschieben des Stopfens (1) in Schritt v) mittels des Aufnahmebehältnisses (8) als Handhabe erfolgt.

11. Verfahren nach einem der Ansprüche 9 oder 10,
**dadurch gekennzeichnet,**
**dass** die Schritte iii) bis vi) nach Schritt vi) einmal oder mehrmals wiederholt werden, wobei in Schritt iv) jeweils ein neues, noch unbenutztes Aufnahmebehältnis (8) verwendet wird.

## Claims

1. A phase separator (14) for liquid mixtures, in particular for human blood, having a stopper (1) having a passage (6), a vessel (4) having an opening (2) on one side, a bottom (15) on the other side and an inner wall (3), the opening (2) of which is closed by the stopper (1) by means of a press fit with the inner wall (3), a receptacle (8) and a membrane (5) which closes the passage (6), which, upon manual displacement of the stopper (1) along the inner wall (3) in the direction of the bottom (15) into a liquid located in the vessel (4), is permeable to the liquid through a puncture produced by means of a cannula (9) provided for filling with the liquid mixture to be separated,
**characterized in that,**
the passage (6) on one side of the membrane (5) has a nozzle-like neck (7) for coupling the receptacle (8), the membrane is arranged between the neck (7) and the bottom (15) and the receptacle (8) is provided as a handle for the manual displacement of the stopper (1) and is configured as a piston syringe (13) that can be inserted wholly or partially into the vessel (4) with an outlet (12) that can be coupled to the neck (7), a piston (18) and a syringe body (17), the piston (18) of which can be moved out of the syringe body (17) by the liquid flowing through the puncture.

2. The phase separator (14) according to claim 1,
**characterized in that,**
that the membrane (5), due to its material elasticity, after removing the cannula (9) used for the puncture, closes when the stopper (1) is at rest and opens again so that the liquid can pass through when the stopper (1) is manually displaced along the inner wall (3) in the direction of the bottom (15) into the liquid located in the vessel (4) by means of the receiving vessel (8) as a handle.

3. The phase separator (14) according to any one of the preceding claims,
**characterized in that,**
that the stopper (1) has a lower density than human blood plasma.

4. The phase separator (14) according to any one of the preceding claims,
**characterized in that,**
that the stopper (1) has one or more sealing lips (10) for the press fit.

5. The phase separator (14) according to any one of the preceding claims,
**characterized in that,**
that the vessel (4) is designed as a centrifugation vessel having a protective cap (21) placed on the opening (2), wherein the protective cap (21) is penetrable by means of the cannula (9).

6. The phase separator (14) according to claim 5,
**characterized in that,**
that the protective cap (21) and the stopper (1) are detachably connected to one another, wherein the displaceability of the stopper (1) is provided only after the connection has been released.

7. A method for phase separation of a liquid mixture (19) and subsequent separation of a liquid supernatant (20) by means of a phase separator (14) according to any one of claims 1 to 6 and a cannula (9),
**characterized by the following steps:**
i) penetrating the membrane (5) with the cannula (9), wherein the liquid mixture to be separated flows through the cannula (9) into the vessel (4);
ii) removing the cannula (9) from the membrane (5);
iii) formation of the supernatant to be separated (20);
iv) connecting the receptacle (8) to the neck (7), wherein the receptacle (8) is designed as a piston syringe (13);
v) displacing of the stopper (1) in the direction of the bottom (15) until the liquid supernatant (20) to be separated has penetrated through the membrane (5), wherein the receptacle (8) is introduced wholly or partly into the vessel (4) and the piston (18) of the piston syringe (13) is pressed out of the syringe body (17) by the separated liquid supernatant (20).

8. The method according to claim 7,
**characterized in that,**
that the liquid supernatant (20) to be separated is formed in step iii) by means of centrifugation.

9. The method according to one of claims 7 or 8,
which **comprises the following step** after step v):
vi) uncoupling the receptacle (8).

10. The method according to any one of claims 7 to 9,
**characterized in that,**
that the plug (1) is displaced in step v) by means of the receptacle (8) as a handle.

11. The method according to one of claims 9 or 10,
**characterized in that,**
that steps iii) to vi) are repeated one or more times after step vi), wherein a new, as yet unused receptacle (8) is used in step iv).

## Revendications

1. Séparateur de phases (14) destiné à des mélanges liquides, notamment à du sang humain, pourvu d'un obturateur (1) comportant un passage (6), d'un récipient (4) comportant un orifice (2) sur un côté d'extrémité, un fond inférieur (15) sur l'autre côté d'extrémité et une paroi intérieure (3), dont l'orifice (2) est fermé par l'obturateur (1) par ajustement serré avec la paroi intérieure (3), d'un contenant récepteur (8) et d'une membrane (5) fermant le passage (6), qui lors d'un déplacement manuel de l'obturateur (1) le long de la paroi intérieure (3) dans la direction du fond inférieur (15) dans un liquide se trouvant dans le récipient (4), est perméable audit liquide, à travers un perçage généré au moyen d'une canule (9) prévue pour un remplissage avec le mélange liquide qui doit être séparé,
**caractérisé en ce que**
le passage (6) comporte sur un côté de la membrane (5) un embout (7) de type tubulure, destiné à l'accouplement du contenant récepteur (8), la membrane est placée entre l'embout (7) et le fond inférieur (15) et le contenant récepteur (8) est prévu comme manette, pour le déplacement manuel de l'obturateur (1) et qui en tant que seringue à piston (13) susceptible d'être introduite totalement ou partiellement dans le récipient (4) est conçue avec une sortie (12), susceptible d'être accouplée sur l'embout (7), un piston (18) et un corps de seringue (17), dont le piston (18) est susceptible d'être déplacé hors du corps de seringue (17) par le liquide circulant à travers le perçage.

2. Séparateur de phases (14) selon la revendication 1,
**caractérisé en ce que**
du fait de l'élasticité de sa matière, après le retrait de la canule (9) prévue pour le perçage, la membrane (5) se ferme au repos de l'obturateur (1) et lors d'un déplacement manuel de l'obturateur (1) le long de la paroi intérieure (3) en direction du fond inférieur (15) dans le liquide se trouvant dans le récipient (4) par l'intermédiaire du contenant récepteur (8) s'ouvre à nouveau, en tant que manette, suffisamment pour permettre au liquide de passer à travers elle.

3. Séparateur de phases (14) selon l'une quelconque des revendications précédentes,
**caractérisé en ce que**
l'obturateur (1) fait preuve d'une densité inférieure à celle du plasma sanguin humain.

4. Séparateur de phases (14) selon l'une quelconque des revendications précédentes,
**caractérisé en ce que**
l'obturateur (1) comporte une ou plusieurs lèvres d'étanchéité (10) pour l'ajustement serré.

5. Séparateur de phases (14) selon l'une quelconque des revendications précédentes,
**caractérisé en ce que**
le récipient (4) est réalisé sous la forme d'un récipient de centrifugation, pourvu d'un capuchon protecteur (21) placé sur l'orifice (2), le capuchon protecteur (21) étant susceptible d'être percé au moyen de la canule (9).

6. Séparateur de phases (14) selon la revendication 5,
**caractérisé en ce que**
le capuchon protecteur (21) et l'obturateur (1) sont assemblés l'un à l'autre de manière amovible, l'aptitude au déplacement de l'obturateur (1) n'étant donnée qu'après désolidarisation de l'assemblage.

7. Procédé, destiné à séparer les phases d'un mélange (19) liquide et à séparer ensuite un surnageant (20) liquide au moyen d'un séparateur de phases (14) selon l'une quelconque des revendications 1 à 6 et d'une canule (9),
**caractérisé par les étapes suivantes, consistant à** :
i) percer la membrane (5) à l'aide de la canule (9), le mélange liquide qui doit être séparé s'écoulant à travers la canule (9) dans le récipient (4) :
ii) retirer la canule (9) hors de la membrane (5) ;
iii) créer le surnageant (20) qui doit être séparé ;
iv) assembler le contenant récepteur (8) avec l'embout (7), le contenant récepteur (8) étant conçu sous la forme d'une seringue à piston (13) ;
v) déplacer l'obturateur (1) dans la direction du fond inférieur (15) jusqu'à ce que le surnageant (20) liquide soit poussé à travers la membrane (5), le contenant récepteur (8) étant introduit totalement ou partiellement dans le récipient (4) et le piston (18) de la seringue à piston (13) étant poussé à travers le surnageant (20) liquide hors du corps de seringue (17).

8. Procédé selon la revendication 7,
**caractérisé en ce que**
la création du surnageant (20) liquide qui doit être séparé s'effectue dans l'étape iii) au moyen d'une centrifugation.

9. Procédé selon l'une quelconque des revendications 7 ou 8,
qui après l'étape v) comprend **l'étape suivante** :
vi) désaccouplement du contenant récepteur (8).

10. Procédé selon l'une quelconque des revendications 7 à 9,
**caractérisé en ce que**
le déplacement de l'obturateur (1) dans l'étape v) s'effectue au moyen du contenant récepteur (8) en tant que manette.

11. Procédé selon l'une quelconque des revendications 9 ou 10,
**caractérisé en ce que**
les étapes iii) à vi) sont répétées une fois ou plusieurs fois après l'étape vi), dans l'étape iv) étant utilisé chaque fois un nouveau contenant récepteur (8) encore inutilisé
